# EUROPEAN PATENT APPLICATION

(11) **EP 1 716 860 A1**
(43) Date of publication of application: **02.11.2006**
(21) Application number: 05710288.1
(22) Date of filing: 09.02.2005
(51) Int. Cl.: A61K 38/00, A61P 11/00

(54) **PREVENTIVE AND THERAPEUTIC AGENT FOR CHRONIC OBSTRUCTIVE PULMONARY DISEASE**

(30) Priority: 13.02.2004 JP 2004346572
(71) Applicant: Redox Bioscience Inc., Kyoto-shi, Kyoto 606-8396 (JP)
(72) Inventor: YODOI, Junji, Kyoto-shi, Kyoto 606-8255 (JP); SON, Aoi, Kyoto-shi, Kyoto 615-0835 (JP); NAKAMURA, Hajime, Hirakata-shi, Osaka 573-1118 (JP)
(74) Representative: Copp, David Christopher
(86) International application number: PCT/JP2005/002388
(87) International publication number: WO 2005/077399

(57) **Abstract**

[Problems]

To provide a preventive and therapeutic agent of high efficacy with reduced side effects for chronic obstructive pulmonary disease.

[Means for solving problems]

There is provided a preventive and therapeutic agent for chronic obstructive pulmonary disease, comprising a thioredoxin superfamily polypeptide as an active ingredient.

## Description

### BACKGROUND OF THE INVENTION

### Technical Field

The present invention relates to a preventive or therapeutic agent for chronic obstructive pulmonary disease, and particularly to an agent including polypeptides of the thioredoxin superfamily (referred to below as TRXs) for preventing or treating chronic obstructive pulmonary disease.

### Description of the Related Art

Air pollutants, such as fine particles in tobacco smoke, fine particles in exhaust gas, dust, and fine particles in soot are considered as a cause of inflammation, fibrosis, and necrosis in the respiratory organs (e.g. lungs), and in respiratory cells. Such inflammation, fibrosis, and necrosis may lead to pulmonary disease, such as Chronic Obstructive Pulmonary Disease (referred to below as COPD).

Among the air pollutants that cause pulmonary diseases, Diesel Exhaust Particle (referred to below as DEP), in particular, has been a subject of many researches about its effect on the lungs.

DEP is fine particles made of carbon particles surrounded by chemical substances generated by diesel combustion. Such fine particles are known as one of air pollutants. DEP can be easily taken into the human respiratory organs.

Inhalation of DEP, as well as inhalation of tobacco smoke or of air pollutants, is considered as a cause of inflammation, fibrosis, and necrosis in the respiratory organs, such as lungs, and in respiratory cells. Such inflammation, fibrosis, and necrosis lead to diseases, such as, pulmonary emphysema, chronic bronchitis, and Chronic Obstructive Pulmonary Disease (referred to below as COPD). These diseases are known to be induced by air pollution.

In this regard, researches have been made using model mice with a lung disorder caused by DEP in order to find compounds effective as a preventive or therapeutic agent for COPD.

Effective compounds for lung disorders that DEP causes in model mice are considered to be equally effective for a wide range of lung disorders caused by smoking or by air pollutants (e.g. fine particles in exhaust gas, fine particles in tobacco smoke, dust, and fine particles in soot), such as COPD.

No agents that can effectively prevent or treat lung diseases (e.g. COPD) without causing adverse effects have been reported so far.

Therefore, there is a strong need for developing preventive or therapeutic agents for COPD while causing little adverse effects.

### SUMMARY OF THE INVENTION

It is an object of the present invention to provide an agent for effectively preventing or treating COPD while causing little adverse effects.

The present inventors discovered that TRXs have an effect of preventing infiltration of neutrophils and lymphocytes into lung cells and that TRXs are effective for preventing or treating COPD, and completed the present invention.

The present invention provides a preventive or therapeutic agent for chronic obstructive pulmonary disease containing polypeptide of the thioredoxin superfamily.

In one aspect of the present invention, the chronic obstructive pulmonary disease is accompanied by infiltration of neutrophils and lymphocytes into lung cells.

In one aspect of the present invention, the chronic obstructive pulmonary disease is caused by smoking or by pollutants in the air.

In one aspect of the present invention, the pollutants in the air are selected from a group consisting of fine particles in tobacco smoke, fine particles in exhaust gas, dust, and fine particles in soot.

In one aspect of the present invention, the chronic obstructive pulmonary disease is either pulmonary emphysema or chronic bronchitis.

In one aspect of the present invention, the chronic obstructive pulmonary disease is accompanied by one of the symptoms selected from a group consisting of inflammation, fibrosis, and necrosis in tissues and cells.

In one aspect of the present invention, the polypeptide of the thioredoxin superfamily is human thioredoxin.

The preventive or therapeutic agent containing polypeptide of the thioredoxin superfamily as active ingredients according to the present invention is effective for COPD.

The preventive or therapeutic agent containing polypeptide of the thioredoxin superfamily as an active ingredient according to the present invention strongly inhibits the infiltration of neutrophils and lymphocytes into lung cells. Therefore, the preventive or therapeutic agent of the present invention is effective for COPD with such infiltration.

The preventive or therapeutic agent according to the present invention has little risk of causing adverse effects because its active ingredient is thioredoxin, which is intrinsic thiol protein to be expressed in vivo as well.

### BRIEF DESCRIPTION OF THE DRAWINGS

FIG. 1 shows a lung tissue section (× 12, × 100) from TRX transgenic mice (TRX Tg) and from C57BL/6 mice. DEP was injected into both mice through the trachea.
FIG. 2 shows the steps of the experiment described in example 2.
FIG. 3a shows in contrast the amount of IL-2 produced by mast cells with high expression of TRX and by control cells. B6 indicates control cells while TRX Tg indicates mast cells with high expression of TRX.
FIG. 3b shows in contrast the amount of IL-6 produced by mast cells with high expression of TRX and by control cells. B6 indicates control cells while TRX Tg indicates mast cells with high expression of TRX.
FIG. 4 shows the steps of the experiment described in example 3.
FIG. 5 shows the histamine released from mast cells with high expression of TRX and from control cells. B6 indicates control cells while TRX Tg indicates mast cells with high expression of TRX.

### DESCRIPTION OF THE PREFERRED EMBODIMENTS

Thioredoxin (TRX) is 12 kDa multifunctional peptide that has redox-active disulfide/ dithiol at its active site sequence of -Cys-Gly-Cys-(Redox regulation of cellular activation Ann. Rev. Immunol.1997; 15:351-369).

Since thioredoxin was first isolated from Escherichia coli as a hydrogen-donor to ribonucleotide reductase and as an important enzyme in the synthesis of deoxyribonucleotide, it has been isolated and identified from many prokaryotes and eukaryotes.

ATL-derived factor (ADF) is human thioredoxin that the present inventors first identified as IL-2 receptor inductor produced by HTLV-infected T-lymphocytes.

Intracellular thioredoxin plays an important role in controlling transcription factor associated with redox (transcription factor such as activator protein-1 and nuclear factor κ B) (SeeAP-1 transcriptional activity is regulated by a direct association between thioredoxin and Ref-1 PNAS. 1997;94:3633-3638).

Human thioredoxin controls signal transduction of p38 mitogen activating protein kinase (MAPK) and apotosis signal regulating kinase-1 (ASK-1).

The present inventors have reported that thioredoxin is released extracellularly and expresses cytokine activity or chemokine activity (Circulating thioredoxin suppresses lipopolysaccharide-induced neutrophil chemotaxis PNAS.2001; 98: 15143-15148) and that extracellular TRX shifts intracellularly (Redox-sensing release of thioredoxin from T-lymphocytes with negative feedback loops J. Immunol. 2004;172:442-448).

In the present invention, the present inventors found a new action of thioredoxin including the inhibiting on the infiltration of neutrophils and lymphocytes into lung cells and completed the present invention.

The examples of the present invention will be described below.

The respiratory organs, as used herein, includes all organs related to alveolar gas exchange including nasal cavity, paranasal sinuses, pharynx, larynx, trachea, bronchi, lung, and the like.

Human thioredoxin (hTRX), as used herein, means polypeptide consisting of the 105 amino acids of sequence No. 1.

In addition to human thioredoxin, thioredoxin in the present invention may be any compound belonging to the "thioredoxin family". Such compounds can be, for example, those having at their active center polypeptide with the following amino acid sequences: -Cys-Gly-Pro-Cys-, -Cys-Pro-Tyr-Cys-, -Cys-Pro-His-Cys-, and -Cys-Pro-Pro-Cys-.

Among these compounds, thioredoxin having the sequence -Cys-Gly-Pro-Cys- at its active center or thioredoxin 2 (mitochondria-specific thioredoxin) is preferable.

Human thioredoxin in the present invention may also be derivatives of TRX prepared by a known technique of genetic engineering from hTRX with Sequence No.1.

In the sequence of such derivatives of TRX, one or more amino acids, preferably one or several amino acids, are replaced, lost, added, or inserted in the positions other than either position 32 or position 35 of Sequence No. 1, preferably in the positions other than positions 32-35 of Sequence No. 1. Such derivatives of TRX have an activity of inhibiting the infiltration of nerutrophils and lymphocytes into lung cells and the like.

Sequence No. 2 is the nucleotide sequence of Sequence No. 1.

The present inventors prepared model mice with lung disorders caused by DEP (Diesel Exhaust Particle) from thioredoxin transgenic mice and found thioredoxin transgenic mice suffered much milder lung diseases than wild type mice.

DEP is fine particles made of carbon particles surrounded by chemical substances generated by diesel combustion. Such fine particles are known as one of air pollutants. DEP can be easily taken into the human respiratory organs.

Inhalation of DEP is considered as a cause of inflammation, fibrosis, and necrosis in the respiratory organs (e.g. lungs) and in respiratory cells. Such inflammation, fibrosis, and necrosis lead to diseases such as bronchial asthma, pulmonary emphysema, and chronic bronchitis. These diseases are known to be induced by air pollution.

Effective compounds for lung disorders caused by DEP in model mice are considered to be similarly effective for a wide range of lung disorders caused by air pollutants (e.g. fine particles in exhaust gas, fine particles in tobacco smoke, dust, and fine particles in soot).

Therefore, it is suggested that the preventive or therapeutic agent of the present invention is effective for preventing or treating respiratory diseases caused by air pollutants such as fine particles in tobacco smoke, fine particles in exhaust gas, dust, and fine particles in soot.

Particularly, since TRXs have a function of inhibiting infiltration of neutrophils and lymphocytes into lung cells as described below, TRXs can be preferebly used as a preventive or therapeutic agent for COPD caused by such infiltration.

Furthermore, the present inventors found that TRXs have an inhibiting action on degranulation of mast cells and an inhibiting action on the production (secretion) of cytokines that are released from mast cells and that TRXs are effective as a preventive or therapeutic agent for allergic diseases caused by these cytokines.

The cytokines released from mast cells include, though not limited to these, histamine, IL-2, IL-4, IL-6, GM-CSF, and TNF-α. Among these, histamine, IL-2, and IL-6 have particularly close relationship with allergic diseases. By inhibiting the production (secretion) of these cytokines, preventive or therapeutic effect for allergic diseases can be achieved.

Allergic diseases related to histamine, IL-2, and IL-6 include, though not limited to these, atopic bronchial asthma and allergic rhinitis (e.g. pollinosis).

The preventive or therapeutic agent of the present invention can be administered orally or nonorally. The way of administration may be selected by clinicians.

When the preventive or therapeutic agent of the present invention are orally administered, it may be administered as solid preparations (e.g., tablets, pills, powders, coated tablets, granules, and capsules), liquid preparations (e.g., solutions, suspensions, emulsions, and syrups), inhalant preparations (e.g., aerosols, atomizers, and nebulizers), or liposome-encapsulated preparations.

The preventive or therapeutic agent according to the present invention is practically used as drugs with pharmaceutically tolerable vehicles.

Such pharmaceutical vehicles may be, for example, binders, disintegrators, surfactants, absorption promoters, moisture retainers, adsorbents, lubricants, fillers, volume expanders, moisteners, antiseptics, stabilizers, emulsifiers, solubilizers, salts to control the osmotic pressure, and diluents or fillers such as buffers. These are used selectively according to the dosing units of the agent.

To form the drugs containing the preventive or therapeutic agent of the present invention into tablets, the pharmaceutical vehicles can be, for example, fillers such as lactose, white sugar, sodium chloride, glucose, urea, starch, calcium carbonate, kaolin, crystalline cellulose, silicate, and potassium phosphate; binders such as water, ethanol, propanol, simple syrup, glucose solution, starch suspension, gelatin solution, carboxy methylcellulose, hydroxyl propylcellulose, methylcellulose, and polyvinylpyrrolidone; disintegrators such as carboxy methylcellulose sodium, carboxy methylcellulose calcium, low-grade substituted hydroxy propylcellulose, dry starch, sodium arginate, agar powder, laminaran powder, sodium hydrogen carbonate, and calcium carbonate; surfactants such as polyoxyethylene sorbitan fatty acid esters, sodium lauryl sulfate, and monoglyceride stearate; disintegration inhibitors such as white sugar, stearin, cacao butter, and hydrogen-added oil; absorption promoters such as class 4 ammonium base and sodium lauryl sulfate; moisture retainers such as glycerin and starch; absorbents such as starch, lactose, kaolin, bentonite, and colloid silicate; and lubricants such as purified talc, stearate, boric acid power, and polyethylene glycol.

In addition, such tablets may be, if necessary, coated with a conventional coating. For example, the tablets may be prepared as sugar-coated tablets, gelatin-coated tablets, enteric-coated tablets, or film-coated tablets. The tablets may also be coated with two or more coatings to be double-layered or multi-layered.

To form such drugs into pills, the pharmaceutical vehicles can be, for example, fillers such as glucose, lactose, starch, cacao fat, hardened plant oil, kaolin, and talc; binders such as Arabic gum powder, tragacanth power, gelatin, and ethanol; and disintegrators such as laminaran and agar.

To administer the preventive or therapeutic agent of the present invention non-orally, the agent can be used, for example, in the forms of injection preparation (solutions, emulsions, suspensions, etc.) for intravenous, subcutaneous, intradermal, intramuscular, or intraperitoneal injection, solutions (e.g., eye drops, nasal solutions), suspensions, emulsions, and instillation agent.

When the pharmaceutical agent of the present invention is prepared as injections such as solutions, emulsions, and suspensions, they should preferably be sterilized and adjusted to be isotonic to blood. To prepare the therapeutic or preventive agent of the present invention into such dosage forms, diluting agent may be used. The diluting agent can be, for example, water, ethyl alcohol, macrogol, propylene glycol, ethoxy isostearyl alcohol, polyoxy isostearyl alcohol, and polyoxy ethylene sorbitan fatty acid esters.

In this process, salt, glucose, or glycerin may be added to the pharmaceutical agent of the present invention in amounts sufficient for adjusting the solution to be isotonic to blood.

Common solubilizers, buffers, soothing agent and the like may also be added to the solution.

When the preventive or therapeutic agent of the present invention is provided as liquid agent, it may be preserved after removing water by freezing or by freeze-drying. The freeze-dried agents are redissolved with, for example, distilled water for injection before use.

Furthermore, while preparing the preventive or therapeutic agent of the present invention, the agent may be mixed, if necessary, with coloring agents, preservatives, flavoring agents, seasoning agents, sweetening agents, or other drugs.

The effective dose of polypeptide of the TRX family may be determined easily by those skilled in the art based on conventional techniques. For example, the effective dose for an adult is about 0.1 mg/kg to 10 g/kg, more preferably about 1-10 mg/kg, and most preferably about 1-500 mg/kg.

This daily dose may be administered at one time or in several doses. The daily dose is preferably adjusted according to the drug type, patient's sexuality and age, and severity of the disorder.

Some examples of the present invention will be explained below. The present invention, however, is not to be limited to these examples but many modifications are possible within the scope of the invention using the general knowledge of those skilled in the art.

### Example 1

Model mice with lung disorder caused by DEP were prepared using C57BL/6 mice and TRX transgenic mice (available from Ajinomoto Co., Inc.).

After the mice were anesthetized, they were subjected to intratracheal instillation of DEP through the trachea.

After this, the lungs of these mice were removed to prepare paraffin embedded sections. These sections were then stained with hematoxilin-eosin.

The result showed that slight infiltration of neutrophils and lymphocytes into lung cells was observed in the lung tissue of TRX transgenic mice while significant infiltration was observed in that of C57BL/6 mice.

### Example 2

### Inhibiting effect of thioredoxin on cytokine production of mast cells

### 1. Preparation of cells

C57BL/6 mice and TRX transgenic mice (available from Ajinomoto Co., Inc.) were dissected to obtain the femora. From the femora, 5 mL Bone marrow was collected into a glass syringe containing inside 10 mL culture medium. The culture medium included 15 % inactivated fetal bovine serum, 200 mM L-gultamine, 50 mM β-mercaptoethanol, and RMI containing 100 pM rmIL. The collected bone marrow was poured into a 25 cm² T-flask and cultured there. After one week of cultivation, the cultured bone marrow was moved into a middle-sized flask to which 30 mL culture medium was added. After further two weeks, the cultured bone marrow was moved into a large-sized 175 cm² T-flask and cultured there in 100 mL culture medium. Mast cells were obtained 4-5 weeks after the cultivation was started (Fig. 2).

### 2. Cytokine production ability of mast cells

After suspending 1 × 10⁷ mast cells into 10 mL RPMI culture medium containing 10 % fetal bovine serum, anti-DNP (antidinitrophenil) IgE antibody was added to the culture medium so that its final concentration was 1.2 µg/mL. In this culture medium, the mast cells were then cultivated for 18 hours. The mast cells were collected and washed before the cells were suspended into culture medium so that their final concentration was 1 × 10⁶ cells/mL and then 200 µL of the suspension was added to each hole in a 96-hole plate and was cultivated there. Two or three hours after that, DNP-BSA was added to the culture so that its final concentration was 30 ng/mL. The culture was then further cultivated for 18 hours (Fig. 2). The cytokines (IL-2 and IL-6) contained in the conditioned medium were measured using ELISA method. The result showed that the amount of IL-2 and IL-6 produced from mast cells with high expression of TRX was significantly lower than that from mast cells derived from control mice (See Fig 3a and Fig 3b).

### Example 3

### Inhibiting effect of thioredoxin on degranulation of mast cells

### 1. Preparation of mast cells and sensitization to antigens

After suspending 1 × 10⁷ mast cells into 10 mL RPMI culture medium containing 10 % fetal bovine serum, anti-DNP (antidinitrophenil) IgE antibody was added to the culture medium so that its final concentration was 1-2 *µ*g/mL. In this culture medium, the mast cells were then cultivated for 18 hours. The mast cells were collected and washed before the cells were suspended into culture medium so that their final concentration was 1 × 10⁷ cells/mL and then 200 *µ*L of the suspension was added to each hole in a 96-hole plate and cultivated there. Two or three hours after that, DNP-BSA was added to the culture so that its final concentration was 30 ng/mL. The culture was then further cultivated for 20 minutes. Next, to the resultant culture, 10 µL buffer solution for stopping the reaction (4mM EDTA, 50mM HEPES pH 7.4, 1 % glucose, 1 % gelatin) was added. The culture was then left on ice before the conditioned medium was collected (Fig. 4).

### 2. Quantitative determination on histamine

The conditioned medium that had been collected from the culture of mast cells was added to a Shodex-Asahipak ODP-50 4D (Available from Tosoh Corporation). The column was equilibrated with sodium borate buffer (pH 9.5). In this column, buffer solution was allowed to flow at a rate of 0.5 mL/minute. Then chromatography was carried out to determine the amount of histamine (excitation wavelength: 330 nm; emitting wavelength: 440 nm). The result showed that the amount of IL-2 and IL-6 produced from mast cells with high expression of TRX was lower than that from mast cells derived from control mice (See Fig 5).

## Claims

1. A preventive or therapeutic agent for chronic obstructive pulmonary disease containing polypeptide of the thioredoxin superfamily.

2. The preventive or therapeutic agent of claim 1, wherein said chronic obstructive pulmonary disease is accompanied by infiltration of neutrophils and lymphocytes into lung cells.

3. The preventive or therapeutic agent of either claim 1 or 2, wherein said chronic obstructive pulmonary disease is caused by smoking or by pollutants in the air.

4. The preventive or therapeutic agent of claim 3, wherein said pollutants in the air are selected from a group consisting of fine particles in tobacco smoke, fine particles in exhaust gas, dust, and fine particles in soot.

5. The preventive or therapeutic agent according to any one of claim 1 to claim 4, wherein said chronic obstructive pulmonary disease is either pulmonary emphysema or chronic bronchitis.

6. The preventive or therapeutic agent according to any one of claim 1 to claim 5, wherein said chronic obstructive pulmonary disease is accompanied by one of the symptoms selected from a group consisting of inflammation, fibrosis, and necrosis in tissues and cells.

7. The preventive or therapeutic agent according to any one of claim 1 to claim 6, wherein said polypeptide of the thioredoxin superfamily is human thioredoxin.
